# FASCICULE DE BREVET EUROPEEN

(11) **EP 3 074 522 B1**
(45) Date de publication et mention de la délivrance du brevet: **29.04.2020**
(21) Numéro de dépôt: 14827466.5
(22) Date de dépôt: 28.11.2014
(51) Int. Cl.: C12P 23/00, C12N 1/12, C12P 21/00

(54) **PROCÉDÉ D'ENRICHISSEMENT EN CAROTENOÏDES ET EN PROTÉINES DE LA BIOMASSE DE MICROALGUES**
VERFAHREN ZUR ANREICHERUNG VON MIKROALGENBIOMASSE MIT KAROTINOIDEN UND MIT PROTEINEN
PROCESS FOR ENRICHMENT OF MICROALGAL BIOMASS WITH CAROTENOIDS AND WITH PROTEINS

(30) Priorité: 29.11.2013 FR 1361837
(43) Date de publication de la demande: 05.10.2016
(73) Titulaire: Corbion Biotech, Inc., South San Francisco, CA 94080 (US)
(72) Inventeur: COSSART, Mathieu, F-62540 Marles les Mines (FR); DEFRETIN, Sophie, F-62400 Bethune (FR); MACQUART, Gabriel, F-62350 Mont Bernanchon (FR); SEGUEILHA, Laurent, F-59520 Marquette lez Lille (FR)
(74) Mandataire: Cabinet Becker et Associés
(86) Numéro de dépôt international: PCT/FR2014/053075
(87) Numéro de publication internationale: WO 2015/079182

(56) Documents cités:
- EP-A1- 1 724 357
- US-A- 2 949 700
- US-A- 3 108 402
- US-A- 3 142 135
- SANSAWA H ET AL: "Production of intracellular phytochemicals in Chlorella under heterotrophic conditions", JOURNAL OF BIOSCIENCE AND BIOENGINEERING, ELSEVIER, AMSTERDAM, NL, vol. 98, no. 6, 1 janvier 2004 (2004-01-01), pages 437-444, XP004727088, ISSN: 1389-1723

## Description

La présente invention se rapporte à un procédé d'enrichissement en caroténoïdes et en protéines de la biomasse de microalgues du genre *Chlorella,* plus particulièrement de l'espèce *Chlorella sorokiniana.*

Les algues, macro et micro, ont une richesse spécifique en grande partie inexplorée. Leur exploitation à des fins alimentaire, chimique ou bioénergétique est encore très marginale. Elles recèlent cependant des composants de grande valeur.

Les microalgues sont en effet des sources de vitamines, lipides, protéines, sucres, pigments et antioxydants.

Les algues et microalgues intéressent ainsi le secteur industriel qui les utilise pour la fabrication de compléments alimentaires, d'aliments fonctionnels, de cosmétiques, de médicaments ou pour l'aquaculture.

Les microalgues sont avant tout des microorganismes photosynthétiques qui colonisent tous les biotopes exposés à la lumière.

A l'échelle industrielle, leur culture monoclonale est réalisée dans des photobioréacteurs (conditions autotrophiques : à la lumière avec du CO₂) ou pour certaines d'entre elles, également dans des fermenteurs (conditions hétérotrophiques : à l'obscurité en présence d'une source carbonée).

Quelques espèces de microalgues sont en effet capables de pousser en absence de lumière : *Chlorella, Nitzschia, Cyclotella, Tetraselmis, Crypthecodinium, Schizochytrium.*

Il est d'ailleurs estimé que la culture en conditions hétérotrophiques coûte 10 fois moins chère qu'en conditions phototrophiques car, pour l'Homme du métier, ces conditions hétérotrophiques autorisent :
- l'utilisation de fermenteurs identiques à ceux utilisés pour les bactéries et les levures et permettent le contrôle de tous les paramètres de culture, et
- la production de biomasses en quantité bien plus élevée que ce qui est obtenu par culture basée sur la lumière.

L'exploitation rentable des microalgues nécessite généralement la maîtrise des conditions de fermentation permettant d'accumuler ses composants d'intérêt, tels que :
- les pigments (chlorophylle a, b et c, β-carotène, astaxanthine, lutéine, phycocyanine, xanthophylles, phycoérythrine...) dont la demande est croissante, tant pour leurs remarquables propriétés antioxydantes, que pour leur apport de couleurs naturelles dans l'alimentation,
- les protéines, ceci afin d'en optimiser les qualités nutritives ; ou
- les lipides, ceci afin d'optimiser leur contenu en acides gras (jusqu'à 60 %, voir 80 % en poids de leur matière sèche) notamment pour :
   - les applications biocarburants, mais aussi
   - les applications en alimentation humaine ou animale, lorsque les microalgues sélectionnées produisent des acides gras polyinsaturés ou PUFAs dits "essentiels" (i.e. apportés par l'alimentation car ne sont pas naturellement produits par l'homme ou l'animal).

L'utilisation de microalgues du genre *Chlorella* pour produire des caroténoïdes est ainsi décrite dans l'art antérieur, notamment dans les brevets US2949700, US3142135 et US 3103402 et l'article de Sansawa and Endo (J Biosci Bioeng. 2004;98(6):437-44), de même que l'utilisation de l'algue verte *Haematococcus pluvialis* pour la production d'astaxanthine (EP1724357).

Pour parvenir à ce résultat, de premiers procédés de fermentation permettant d'obtenir de hautes densités cellulaires (acronyme anglais : HCD pour *High-Cell-Density*) ont été ainsi beaucoup travaillés, de manière à obtenir des rendements et productivités maximales en protéines ou en lipides.

L'objectif de ces cultures HCD était l'obtention de la concentration la plus élevée possible du produit souhaité dans le laps de temps le plus court.

Ce précepte se vérifie par exemple pour la biosynthèse d'astaxanthine par *Chlorella zofingiensis,* où la croissance de la microalgue s'est avérée corrélée directement avec la production de ce composé (Wang and Peng, 2008, World J Microbiol. Biotechnol., 24(9), 1915-1922).

Cependant, le fait de maintenir la croissance à son taux maximal (µ, en h⁻¹) n'est pas toujours corrélé avec la production élevée du produit souhaité.

Il est en effet vite apparu aux spécialistes du domaine qu'il faut par exemple soumettre les microalgues à un stress nutritionnel qui limite leur croissance lorsqu'on souhaite leur faire produire d'importantes réserves lipidiques.

Il est donc procédé désormais au découplage croissance / production dans les procédés fermentaires.

Par exemple, pour favoriser l'accumulation d'acides gras polyinsaturés (ici l'acide docosahexaénoïque ou DHA), la demande de brevet WO 01/54510 recommande de dissocier la croissance cellulaire et la production d'acides gras polyinsaturés.

Chez la microalgue *Schizochytrium sp* souche ATCC 20888, il est ainsi procédé à une première phase de croissance sans limitation en oxygène, de manière à favoriser l'obtention d'une haute densité cellulaire (plus de 100 g/l) puis, dans une deuxième phase, de ralentir progressivement l'apport en oxygène, afin de stresser la microalgue, ralentir sa croissance et enclencher la production des acides gras d'intérêt.

Chez la microalgue *Crypthecodinium cohnii,* la teneur la plus élevée en acide docosahexaénoïque (DHA, acide gras polyinsaturé) est obtenue à faible concentration en glucose (de l'ordre de 5 g/l), et ainsi à faible taux de croissance (Jiang and Chen, 2000, Process Biochem., 35(10), 1205-1209).

Ces résultats illustrent bien le fait que les cinétiques de formation des produits peuvent aussi bien être associées de façon positive que de façon négative avec la croissance des microalgues, voire même avec une combinaison des deux.

De ce fait, dans les cas où la formation des produits n'est pas corrélée avec une croissance cellulaire élevée, il est judicieux de maîtriser le taux de croissance cellulaire.

En général, l'homme du métier choisit de contrôler la croissance des microalgues par la maîtrise des conditions de fermentation (Tp, pH...), ou par l'alimentation régulée en composants nutritionnels du milieu de fermentation (conditions semi continues dites « fed-batch »).

S'il choisit de contrôler la croissance des microalgues en hétérotrophie par l'apport en sources carbonées, l'homme du métier choisit généralement d'adapter la source carbonée (glucose pur, acétate, éthanol...) à la microalgue (C. *cohnii, Euglena gracilis...*) en fonction du métabolite produit (par exemple un acide gras polyinsaturé de type DHA).

La température peut être également un paramètre clef :
- il a par exemple été rapporté que la synthèse d'acides gras polyinsaturés chez certaines espèces de microalgues, tel que l'EPA par *Chlorella minutissima,* est favorisée à une plus basse température que celle requise pour la croissance optimale de ladite microalgue;
- au contraire le rendement en lutéine est plus élevé chez *Chlorella protothecoides* cultivée en hétérotrophie, lorsque l'on augmente la température de production de 24 à 35°C.

*Chlorella protothecoides* est justement reconnue comme une des meilleures microalgues productrices d'huile.

En conditions hétérotrophiques, elle transforme rapidement les hydrates de carbone en triglycérides (plus de 50 % de sa matière sèche).

Pour optimiser cette production en triglycérides, l'homme du métier est amené à optimiser le flux carboné vers la production d'huile, en agissant sur l'environnement nutritionnel du milieu de fermentation.

Il est ainsi connu que l'accumulation en huile se produit lors d'un apport carboné suffisant, mais dans des conditions de carence en azote.

Le rapport C/N est donc ici déterminant, et il est admis que les meilleurs résultats sont obtenus en agissant directement sur la teneur en azote, la teneur en glucose n'étant pas limitante.

De manière non surprenante, cette carence en azote affecte la croissance cellulaire, ce qui résulte en un taux de croissance de 30 % plus faible que le taux de croissance normal de la microalgue (Xiong et al., Plant Physiology, 2010, 154, pp1001-1011) .

Pour expliquer ce résultat, Xiong et al, dans l'article cité supra, démontrent en effet que si l'on divise la biomasse de *Chlorella* en ses 5 composants principaux, i.e. hydrates de carbone, lipides, protéines, ADN et ARN (représentant 85 % de sa matière sèche), si le rapport C/N n'a aucun impact sur la teneur en ADN, ARN et hydrates de carbone, il devient prééminent pour le contenu en protéines et en lipides.

C'est ainsi que les cellules de Chlorelles cultivées avec un rapport C/N faible contiennent 25,8 % de protéines et 25,23 % en lipides, tandis qu'un rapport C/N élevé permet la synthèse de 53,8 % de lipides et 10,5 % de protéines.

Pour optimiser sa production en huile, il est donc primordial pour l'homme du métier de contrôler le flux carboné en le déviant vers la production d'huile, au détriment de la production des protéines ; le flux carboné est redistribué et s'accumule en substances de réserve lipidiques quand les microalgues sont placées en milieu carencé en azote.

Fort de cet enseignement, pour la production de biomasses riches en protéines, l'homme du métier est donc amené à prendre l'opposé de ce contrôle métabolique, i.e. travailler les conditions de fermentation en favorisant plutôt un rapport C/N faible, et ainsi :
- réaliser un apport important en source d'azote au milieu de fermentation, tout en maintenant constant la charge en source carbonée qui sera convertie en protéines et
- stimuler la croissance de la microalgue.

Il s'agit de modifier le flux carboné vers la production de protéines (et donc de biomasses), au détriment de la production des lipides de réserve.

Dans le cadre de l'invention, la société Demanderesse a choisi d'explorer une voie originale en proposant une solution alternative à celle classiquement envisagée par l'homme du métier.

L'invention porte ainsi sur un procédé d'enrichissement en caroténoïdes et en protéines d'une biomasse de microalgue cultivée en hétérotrophie, microalgue du genre *Chlorella,* plus particulièrement encore *Chlorella sorokiniana,* procédé de culture hétérotrophique qui comporte une culture de ladite microalgue dans un milieu minimum, complémenté en une source d'azote qui est un extrait de levures.

Au sens de l'invention, « l'enrichissement » :
- en caroténoïdes : s'entend d'une augmentation de la teneur en caroténoïdes de la biomasse d'au moins 0,05 %, de préférence d'au moins 0,1 % en poids total de la biomasse par rapport à la teneur en caroténoïdes de la biomasse cultivée uniquement dans le milieu minimum. De préférence, la biomasse obtenue par le procédé selon l'invention a une teneur en caroténoïdes d'au moins 0,35 % en poids total de la biomasse, de manière plus particulièrement préférée d'au moins 0,4% en poids total de la biomasse.
- en protéines : s'entend d'une augmentation de la teneur en protéines de la biomasse d'au moins 5 %, de préférence d'au moins 10 % en poids total de la biomasse par rapport à la teneur en protéines de la biomasse cultivée uniquement dans le milieu minimum. De préférence, la biomasse obtenue par le procédé selon l'invention a une teneur en protéines d'au moins 45 % en poids total de la biomasse, de manière plus particulièrement préférée d'au moins 50% en poids total de la biomasse.

Au sens de l'invention, le « milieu minimum » se définit classiquement comme un milieu ne comportant que les éléments chimiques strictement nécessaires à la croissance de la microalgue, sous une forme utilisable par les microalgues n'ayant pas d'exigence particulière.

Le milieu minimum contient alors :
- une source de carbone et d'énergie : généralement du glucose
- une source de potassium et de phosphore : par exemple K₂HPO₄
- une source d'azote et de soufre : par exemple (NH₄)₂SO₄
- une source de magnésium : par exemple MgSO₄.7H₂O
- une source de calcium : par exemple CaCl₂.2H₂O
- une source de fer : par exemple FeSO4.7H₂O
- des sources d'oligoéléments : sels de Cu, Zn, Co, B, Mn, Mo
- des sources de vitamines (thiamine, biotine, vitamine B12...).

La société Demanderesse a alors trouvé que si la culture de microalgue du genre *Chlorella,* plus particulièrement encore *Chlorella sorokiniana,* dans ce milieu minimum, essentiellement minéral, permettait toujours de produire une biomasse importante, cela se faisait au détriment de composants d'intérêt, tels les caroténoïdes et les protéines.

Le taux élevé de la croissance de la biomasse (plus de 0,05 h⁻¹) en milieu essentiellement minéral traduit notamment l'autotrophie de la souche en azote.

Sans être lié par une quelconque théorie, la société Demanderesse a alors émis l'hypothèse selon laquelle une culture de microalgues en milieu minimum orientait les voies métaboliques vers la production de substances de réserve (de type polysaccharides).

La société Demanderesse a alors trouvé que l'apport, en petite quantité (de préférence l'apport azoté reste minéral à plus de 90 %) d'un complément nutritionnel azoté sous forme organique, c'est-à-dire sous la forme d'extraits de levure, dans ces conditions particulières (alors que la microalgue est parfaitement autotrophe pour l'azote) permettait de ralentir la production desdites substances de réserve polysaccharidiques et de détourner les voies métaboliques vers la production de caroténoïdes et de protéines.

Cette conduite de fermentation permet alors de gérer aisément, en milieu minimum, l'ajout des compléments nutritionnels azotés qui augmentent la production de caroténoïdes et de protéines.

Cette stratégie va donc bien à l'encontre du préjugé technique selon lequel pour par exemple augmenter la teneur en protéines de la biomasse, il faut immanquablement augmenter cette biomasse et donc la croissance cellulaire, ou doper le milieu de fermentation en sources d'azote.

En effet, la quantité de biomasse reste ici constante, et c'est l'ajout des compléments nutritionnels (de préférence moins de 10 % en poids de l'azote total ajouté au milieu de fermentation) qui conduit à la surproduction en protéines.

Ainsi, la présente invention concerne un procédé d'enrichissement en caroténoïdes et en protéines d'une biomasse de microalgue cultivée en hétérotrophie, le procédé de culture hétérotrophique comprenant la culture de ladite microalgue dans un milieu minimum complémenté en une source d'azote sous forme organique.

La microalgue est du genre *Chlorella,* en particulier une microalgue riche en pigments choisie parmi *Chlorella sorokiniana, Chlorella vulgaris* et *Chorella kessleri,* et de manière plus particulièrement préférée *Chlorella sorokiniana,*

La source d'azote sous forme organique est un extrait de levure. De préférence, l'extrait de levure est obtenu à partir de *Saccharomyces cerevisiae.*

La source d'azote sous forme organique est ajoutée au milieu minimum comprenant une source d'azote minérale. L'apport d'azote sous forme organique ne dépasse pas 10% de l'azote total contenu dans le milieu de fermentation (sources minérales et organiques cumulées).

La source minérale d'azote dans le milieu minimum peut être par exemple (NH₄)₂SO₄ ou NH₄Cl.

Le milieu minimum peut être complémenté avec 0.5 à 3 g/L d'extrait de levure, de préférence 1 à 2 g/L d'extrait de levure. De manière plus particulièrement préférée, le milieu minimum est complémenté avec environ 1 g/L d'extrait de levure. Tel qu'utilisé ici, le terme « environ » se réfère à une valeur +/- 20%, 10%, 5% ou 2%.

Selon un mode de réalisation, le procédé selon l'invention permet d'augmenter la teneur en protéines de la biomasse d'au moins 5% en poids total de la biomasse par rapport à la teneur en protéines de la biomasse cultivée uniquement dans le milieu minimum. Le procédé selon l'invention peut permettre d'augmenter la teneur en protéines de la biomasse d'au moins 6, 7, 8, 9 ou 10 % en poids total de la biomasse par rapport à la teneur en protéines de la biomasse cultivée uniquement dans le milieu minimum.

De manière préférée, la teneur en protéines dans la biomasse obtenue par le procédé selon l'invention est de plus de 45%, 50% ou 55% en poids total de la biomasse. Selon un autre mode de réalisation, le procédé selon l'invention permet d'augmenter la teneur en caroténoïdes de la biomasse d'au moins 0,05% en poids total de la biomasse par rapport à la teneur en caroténoïdes de la biomasse cultivée uniquement dans le milieu minimum. Le procédé selon l'invention peut permettre d'augmenter la teneur en caroténoïdes de la biomasse d'au moins 0,1 ou 0,2 % en poids total de la biomasse par rapport à la teneur en caroténoïdes de la biomasse cultivée uniquement dans le milieu minimum.

De manière préférée, la teneur en caroténoïdes dans la biomasse obtenue par le procédé selon l'invention est de plus de 0,35, 0,4 ou 0,5 % en poids total de la biomasse.

Selon un autre aspect, la présente invention concerne également un procédé de culture hétérotrophique de microalgues comprenant :
- une première étape de culture des microalgues dans un milieu minimum, et
- une deuxième étape de culture dans laquelle une source d'azote sous forme organique qui est un extrait de levures, est ajoutée au milieu minimum.
La première étape permet la croissance des microalgues et la deuxième étape empêche l'accumulation de substances de réserve polysaccharidiques et permet d'enrichir la biomasse en protéines et caroténoïdes.

La microalgue est du genre *Chlorella,* de préférence choisie parmi *Chlorella sorokiniana, Chlorella vulgaris* et *Chorella kessleri,* et de manière préférée est *Chlorella sorokiniana.*

La présente invention concerne plus particulièrement un procédé de culture hétérotrophique desdites microalgues, notamment *Chlorella sorokiniana,* comprenant:
- une première étape de croissance des microalgues en milieu minimum,
- une deuxième étape dans laquelle de l'extrait de levures est ajouté au milieu minimum.

L'ajout en source d'azote sous forme organique ne dépasse pas 10% de l'azote total contenu dans le milieu de fermentation. En particulier, le milieu minimum peut être complémenté avec 0,5 à 3 g/L d'extrait de levures, de préférence avec 1 à 2 g/L d'extrait de levures.

De préférence, la deuxième étape de culture permet d'augmenter
- la teneur en caroténoïdes de la biomasse d'au moins 0,05 %, de préférence d'au moins 0,1 % en poids total de la biomasse par rapport à la teneur en caroténoïdes de la biomasse cultivée uniquement en milieu minimum, et/ou
- la teneur en protéines de la biomasse d'au moins 5 %, de préférence d'au moins 10 % en poids total de la biomasse par rapport à la teneur en protéines de la biomasse cultivée uniquement en milieu minimum.

Selon un mode préféré, la teneur de la biomasse obtenue
- en caroténoïdes est d'au moins 0,35% en poids total de la biomasse, de préférence d'au moins 0,4% en poids total de la biomasse, et/ou
- en protéines est d'au moins 45% en poids total de la biomasse, de préférence d'au moins 50% en poids total de la biomasse.

De façon optionnelle, comme il sera exemplifié ci-après, dans la deuxième étape, l'apport de glucose est réalisé en continu, à une valeur nettement en dessous de la capacité de consommation du glucose desdites microalgues.

Les modes de réalisation décrits ci-dessus et concernant le procédé d'enrichissement en caroténoïdes et en protéines d'une biomasse de microalgue, sont également envisagés dans cet aspect.

L'invention sera mieux comprise à l'aide des exemples qui suivent, lesquels se veulent illustratifs et non limitatifs.

### Exemple

### Production de C. sorokiniana - ajout d'extrait de levure

La souche utilisée est *Chlorella sorokiniana* UTEX 1663.

### Préculture :

- 600 mL de milieu dans un Erlenmeyer de 2 L ;
- Composition du milieu :

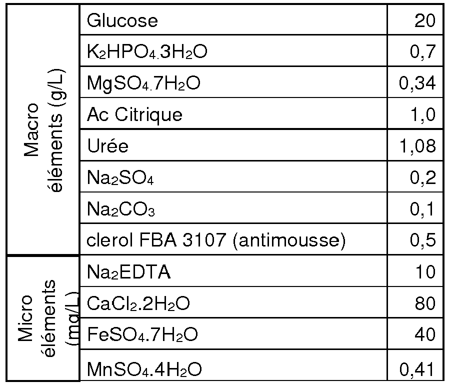

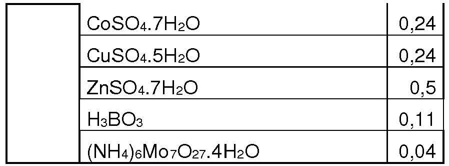

Le pH est ajusté à 7 avant stérilisation par ajout de NaOH 8N

L'incubation se déroule dans les conditions suivantes : durée : 72 h ; température : 28°C ; agitation : 110 rpm (Incubateur Infors Multitron).

La préculture est ensuite transférée dans un fermenteur de 30L de type Sartorius.

### Culture pour production de biomasse :

Le milieu de base est identique à celui de la préculture, mais l'urée est remplacée par du NH₄Cl :

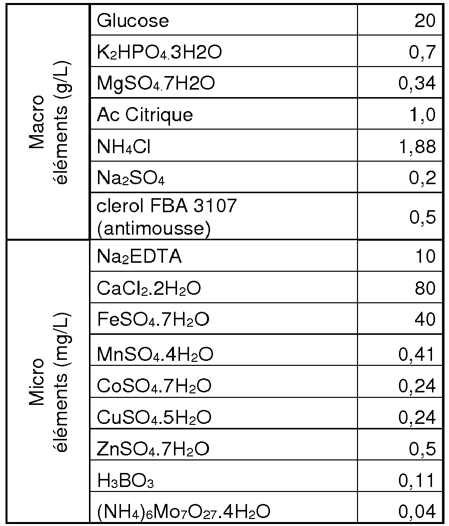

Essai 1 : témoin ; aucun complément nutritionnel n'est ajouté
Essai 2 : 1 g/L d'extrait de levure est ajouté.

Le volume initial (Vi) du fermenteur est ajusté à 13,5 L après ensemencement. Il est porté à 16 - 20 L en final.

Les paramètres de conduite de la fermentation sont les suivants :

| | |
|---|---|
| Température | 28 °C |
| pH | 6,5 - 6,8 par NH₃ 28% w/w |
| pO₂ | > 20% (maintenue par agitation) |
| Agitation | 300 RPM mini |
| Débit d'air | 15 L/min |

Lorsque le glucose apporté initialement est consommé, un apport de milieu similaire au milieu initial est réalisé sous forme d'une solution concentrée contenant notamment 500 g/L de glucose.

Le tableau ci-dessous donne la composition d'un litre de cette solution concentrée :

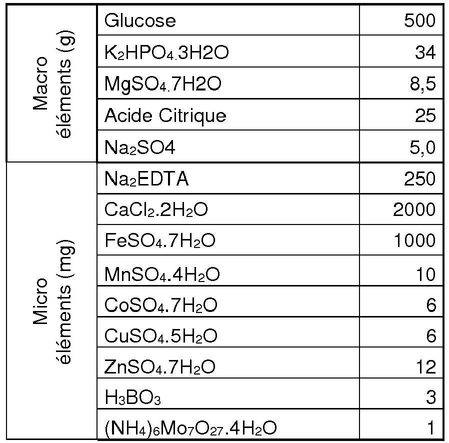

Les concentrations des éléments autres que le glucose ont été déterminées de façon à ce qu'ils soient en excès par rapport aux besoins nutritionnels de la souche.

Cette solution est apportée en continu à une vitesse inférieure à la capacité de consommation de glucose de la souche. De cette manière, la teneur résiduelle en glucose dans le milieu est maintenue nulle, c'est-à-dire que la croissance de la souche est limitée par la disponibilité en glucose (condition glucose-limitant).

Cette vitesse est augmentée au cours du temps de façon exponentielle selon la formule suivante :
S = 12.exp (0,07xt)
*où S = débit d'apport en glucose (en g*/*h) et t = durée du fed-batch (en h)*

De l'antimousse Clerol FBA 3107 est ajouté à la demande pour éviter un moussage excessif.

### Résultats : effet de l'ajout d'extrait de levure

La teneur en protéine de la biomasse obtenue est évaluée par la mesure de l'azote total exprimée en N 6.25.

| Essai | Milieu | Durée (h) | Biomasse (g/L) | % N 6.25 | % Caroténoïdes |
|---|---|---|---|---|---|
| 1 | Base | 72 | 75,7 | 40,0 | 0.3 |
| 2 | Base + 1 g/L d'extrait de levure | 71 | 76,3 | 50,2 | 0.4 |

Ces résultats montrent que le fait d'apporter un complément nutritionnel sous forme d'extrait de levure permet d'obtenir une concentration en biomasse élevée avec une teneur en protéine supérieure à 50%.

La teneur en caroténoïdes est également augmentée.

## Revendications

1. Procédé d'enrichissement en caroténoïdes et en protéines d'une biomasse de microalgue du genre *Chlorella* cultivée en hétérotrophie, comprenant la culture de ladite microalgue dans un milieu minimum complémenté en une source d'azote sous forme organique, dans lequel ladite source d'azote est ajoutée en une quantité telle que l'azote sous forme organique ne dépasse pas 10% de l'azote total contenu dans le milieu de fermentation, et dans lequel la source d'azote sous forme organique est de l'extrait de levures.

2. Procédé selon la revendication 1, **caractérisé en ce que** la microalgue est choisie parmi *Chlorella sorokiniana, Chlorella vulgaris* et *Chorella kessleri,* de préférence est *Chlorella sorokiniana.*

3. Procédé selon l'une quelconque des revendications précédentes, **caractérisé en ce que** le milieu minimum est complémenté avec 0,5 à 3 g/L d'extrait de levures, de préférence avec 1 à 2 g/L d'extrait de levures.

4. Procédé selon l'une quelconque des revendications précédentes, **caractérisé en ce que** la teneur en caroténoïdes de la biomasse obtenue est d'au moins 0,35% en poids total de la biomasse, de préférence d'au moins 0,4% en poids total de la biomasse.

5. Procédé selon l'une quelconque des revendications précédentes, **caractérisé en ce que** la teneur en protéines de la biomasse obtenue est d'au moins 45% en poids total de la biomasse, de préférence d'au moins 50% en poids total de la biomasse.

6. Procédé selon l'une quelconque des revendications précédentes, **caractérisé en ce qu'**il comprend
- une première étape de culture des microalgues dans un milieu minimum, et
- une deuxième étape de culture dans laquelle de l'extrait de levures est ajouté au milieu minimum.

7. Procédé selon l'une quelconque des revendications précédentes, **caractérisé en ce qu'**il comprend :
- une première étape de croissance des microalgues de type *Chlorella sorokiniana* en milieu minimum,
- une deuxième étape de culture dans laquelle de l'extrait de levures est ajouté au milieu minimum.

## Patentansprüche

1. Verfahren zur Anreicherung von Karotinoiden und Proteinen einer Biomasse von heterotroph kultivierten Mikroalgen der Gattung *Chlorella,* umfassend die Kultivierung besagter Mikroalge in einem Minimalmedium, das mit einer organischen Stickstoffquelle supplementiert ist, wobei besagte Stickstoffquelle in einer solchen Menge zugegeben wird, dass der organische Stickstoff nicht 10% des im Fermentationsmedium enthaltenen Gesamtstickstoffs übersteigt, und wobei die organische Stickstoffquelle Hefeextrakt ist.

2. Verfahren gemäß Anspruch 1, **dadurch gekennzeichnet, dass** die Mikroalge aus *Chlorella sorokiniana, Chlorella vulgaris* und *Chlorella kessleri* ausgewählt ist, vorzugsweise *Chlorella sorokiniana* ist.

3. Verfahren gemäß einem der vorhergehenden Ansprüche, **dadurch gekennzeichnet, dass** das Minimalmedium mit 0,5 bis 3 g/l Hefeextrakt, vorzugsweise mit 1 bis 2 g/l Hefeextrakt supplementiert ist.

4. Verfahren gemäß einem der vorhergehenden Ansprüche, **dadurch gekennzeichnet, dass** der Karotinoidgehalt der erhaltenen Biomasse wenigstens 0,35 Gew.-% der gesamten Biomasse, vorzugsweise wenigstens 0,4 Gew.-% der gesamten Biomasse beträgt.

5. Verfahren gemäß einem der vorhergehenden Ansprüche, **dadurch gekennzeichnet, dass** der Proteingehalt der erhaltenen Biomasse wenigstens 45 Gew.-% der gesamten Biomasse, vorzugsweise wenigstens 50 Gew.-% der gesamten Biomasse beträgt.

6. Verfahren gemäß einem der vorhergehenden Ansprüche, **dadurch gekennzeichnet, dass** es umfasst
- einen ersten Schritt der Kultivierung der Mikroalgen in einem Minimalmedium, und
- einen zweiten Schritt der Kultivierung, in dem der Hefeextrakt zu dem Minimalmedium gegeben wird.

7. Verfahren gemäß einem der vorhergehenden Ansprüche, **dadurch gekennzeichnet, dass** es umfasst:
- einen ersten Schritt des Wachstums der Mikroalgen des Typs *Chlorella sorokiniana* in Minimalmedium,
- einen zweiten Schritt, in dem der Hefeextrakt zu dem Minimalmedium gegeben wird.

## Claims

1. A method for carotenoid enrichment and protein enrichment of a heterotrophically cultivated biomass of a microalga of the Chlorella genus, comprising culturing said microalga in a minimum medium supplemented with a nitrogen source in organic form, wherein that said nitrogen source is added in an amount such that nitrogen in organic form does not exceed 10% of the total nitrogen contained in the fermentation medium, and wherein the nitrogen source in organic form is yeast extract.

2. The method according to claim 1, **characterized in that** the microalga is chosen from *Chlorella sorokiniana, Chlorella vulgaris* and *Chorella kessleri,* preferably is *Chlorella sorokiniana.*

3. The method according to any one of the preceding claims, **characterized in that** the minimum medium is supplemented with 0.5 to 3 g/l of yeast extract, preferably with 1 to 2 g/l of yeast extract.

4. The method according to any one of the preceding claims, **characterized in that** the content of carotenoids in the biomass obtained is at least 0.35% by total weight of the biomass, preferably at least 0.4% by total weight of the biomass.

5. The method according to any one of the preceding claims, **characterized in that** the content of proteins in the biomass obtained is at least 45% by total weight of the biomass, preferably at least 50% by total weight of the biomass.

6. The method according to any one of the preceding claims, **characterized in that** it comprises
- a first step of culturing the microalgae in a minimum medium, and
- a second culturing step in which yeast extract is added to the minimum medium.

7. The method according to any one of the preceding claims, **characterized in that** it comprises:
- a first step of growing the microalgae of *Chlorella sorokiniana* type in minimum medium,
- a second culturing step in which yeast extract is added to the minimum medium.
